Europäisches Patentamt

European Patent Office

Office européen des brevets

(19)

(11) Veröffentlichungsnummer : **0 420 042 B1**

(12) **EUROPÄISCHE PATENTSCHRIFT**

(45) Veröffentlichungstag der Patentschrift :
**15.07.92 Patentblatt 92/29**

(21) Anmeldenummer : **90118101.6**

(22) Anmeldetag : **20.09.90**

(51) Int. Cl.⁵ : **A61K 31/275, A61K 9/16**

(54) **Feste Arzneiform mit hohem Verapamil-Gehalt.**

(30) Priorität : **28.09.89 DE 3932378**

(43) Veröffentlichungstag der Anmeldung :
**03.04.91 Patentblatt 91/14**

(45) Bekanntmachung des Hinweises auf die
Patenterteilung :
**15.07.92 Patentblatt 92/29**

(84) Benannte Vertragsstaaten :
**AT BE CH DE DK ES FR GB GR IT LI NL SE**

(56) Entgegenhaltungen :
**EP-A- 0 063 266**
**EP-A- 0 217 778**
**FR-A- 2 620 332**
**R. VOIGT: "Lehrbuch der pharmazeutischen**
**Technologie", Auflage 5, 1984, Seiten 158-162,**
**Kapitel 8, VEB Verlag Vall undGesundheit,**
**Berlin, DE**

(73) Patentinhaber : **KNOLL AG**
**Knollstrasse 32**
**W-6700 Ludwigshafen (DE)**

(72) Erfinder : **Fricke, Helmut, Dr.**
**Pfalzring 159**
**W-6704 Mutterstadt (DE)**
Erfinder : **Moest, Thomas, Dr.**
**An der Duene 9**
**W-2082 Moorrege (DE)**
Erfinder : **Flaig, Ernst, Dr.**
**Langgewann 38/2**
**W-6900 Heidelberg (DE)**

(74) Vertreter : **Karau, Wolfgang, Dr. et al**
**BASF Aktiengesellschaft Carl-Bosch-Strasse**
**38**
**W-6700 Ludwigshafen (DE)**

EP 0 420 042 B1

## Beschreibung

Die Erfindung betrifft feste Arzneiformen mit deutlich höherem Verapamilhydrochlorid-Gehalt als bisher möglich, und Verfahren zu deren Herstellung.

In der Überschrift und im fogenden ist mit "Verapamil" stets Verapamilhydrochlorid gemeint. Man kann natürlich ebensogut andere pharmakologisch unbedenkliche Salze einsetzen. Für sie gelten in den Ansprüchen entsprechend geänderte Mengen.

Handelsübliche Verapamil-Tabletten und -Filmtablettenkerne werden mit einem maximalen Gehalt von 70 % Verapamil hergestellt. Die höchste beschriebene Konzentration liegt bei knapp 80 % (EP-A-0 217 778). Höhere Konzentration können nicht erreicht werden, da Verapamil bei der Verfahrensstufe Granulieren, die eine für das Tablettieren notwendige Voraussetzung ist, zum Schmieren in den Granuliergeräten neigt, und auch nach Trocknung schwer tablettierbar ist, da diese Schmiertendenz auch zum Verkleben in der Matrize der Tablettenpresse führt. Eine Direkttablettierung von handelsüblichem Verapamil in hoher Konzentration (mehr als 70 %) ohne vorheriges Granulieren scheitert an der schlechten Fließfähigkeit des handelsüblichen Rohstoffes, an der mangelhaften Bindefähigkeit trotz Zugabe gut verformbarer Hilfsstoffe wie mikrokristalliner Cellulose sowie an der Tendenz zum Schmieren und Kleben bei hohen Preßdrücken.

Aufgrund der erforderlichen relativ hohen Mengen an Wirkstoff und Hilfsmittelzusätzen sind Verapamil-Tabletten - insbesondere Retard-Tabletten mit ihrem zusätzlichen retardierenden Überzug - relativ groß und dadurch unangenehm zu schlucken.

Der Erfindung lag daher die Aufgabe zugrunde, kleinere Verapamil-Tabletten - insbesondere kleinere Verapamil-Retard-Tabletten - oder auch kleinere, mit Pellets gefüllte Kapseln, die jeweils die gleiche Wirkstoffmenge enthalten, zu entwickeln. Dazu mußte ein Verfahren zur Herstellung eines gut fließfähigen, gleichmäßig feinkörnigen und gut tablettierbaren Granulates und von Pellets mit jeweils hoher Verapamil-Konzentration gefunden werden.

Die Lösung dieser Aufgabe besteht in festen Arzneiformen mit einem Verapamil-Gehalt von mindestens 90, vorzugsweise mindestens 95 und insbesondere mindestens 98 Gew.% und Verfahren zu ihrer Herstellung. Feste Arzneiformen im Sinne dieser Erfindung sind Granulate, Pellets, Tabletten und Filmtabletten. Im Fall von Filmtabletten beziehen sich die vorstehenden Konzentrationsangaben auf den Tablettenkern (ohne Überzug).

Überraschend wurde gefunden, daß ein Verapamil-Granulat mit vorzüglicher Tablettierbarkeit und exzellenter Fließfähigkeit ohne oder mit geringen Hilfsstoffzusätzen und daher hohem Wirkstoffgehalt von bis zu 100 % in einfacher Weise hergestellt werden kann, indem man das Granulieren unter Temperaturerhöhung auf 30 bis 55, vorzugsweise 40 bis 50°C nur mit Wasser (2 bis 10, vorzugsweise 3 bis 7 Gew.-%) oder mit Wasser und geringen Retardier- und/oder Bindemittelzusätzen vornimmt, und daß man bei Wiederholung der Prozedur porenarme Pellets mit glatter Oberfläche und 0,3 bis 1,5 mm Durchmesser erhält, wobei der mittlere Durchmesser und gleichzeitig mindestens 70 Gew.-% im Bereich von 0,5 bis 1 mm liegen.

Die obere Temperaturgrenze ist dadurch gegeben, daß die Mischung zu erweichen beginnt. Bei tieferen Temperaturen als 30 bis 40°C verliert sich der erfindungsgemäße Effekt allmählich. Der erforderliche Energieeintrag Sollte vorzugsweise allein mechanisch erfolgen, kann aber auch durch Beheizen oder Eintrag von warmem (statt kaltem) Wasser unterstützt werden.

Für den Fachmann unerwartet zeigte sich, daß sich beim Granulieren von handelsüblichem Verapamil-Pulver unter Erwärmen ein erheblich geringerer Verbrauch an wäßrigem Granuliermedium (Wasser oder wäßrige Hilfsstoff-Suspension oder -lösung) ergibt als bei Normaltemperatur. Die Verdichtung im pharmaüblichen Granuliermischer, vorzugsweise Hochleistungsgranulator (schnellaufendes Mischelement, ggf. mit Zerhacker, z.B. Lödige-Vertikal- und -Horizontal-Granuliergeräte, Diosna-, Colette-, Henschel-, Fielder-Granulatoren) verläuft dabei entscheidend intensiver als bei Raumtemperatur und ohne Schmieren der Substanz; das Granulat wird bezüglich Korngröße und Korngrößenverteilung fein und homogen, so daß mindestens 70 Gew.-% in einer Korngrößen-Bandbreite von 430 μm innerhalb eines Korngrößenbereichs von 0,1 bis 1 mm, vorzugsweise 0,2 bis 1 mm liegen. Das Auflockern (Egalisieren durch Sieben) des in üblicher Weise (vgl. Handbücher der Pharmazeutischen Technologie) bei 30 bis 50°C auf eine Restfeuchte von 0,5 % oder weniger getrockneten Granulates verläuft unproblematisch ohne Schmiertendenz. Auch feines Granulat (mindestens 80 Gew.-% unter 500 μm) ohne hohen Staubanteil (maximal 10 % unter 100 μm) kann nach dem erfindungsgemäßen Verfahren sehr einfach erzeugt werden. Außerdem ist das Granulat ausgezeichnet rieselfähig, so daß mindestens 250, vorzugsweise mindestens 300 g/min durch den DIN-Becher Nr. 8 hindurchlaufen. Somit ist es mit dem erfindungsgemäßen Granulat möglich, auch kleine Preßlinge herzustellen, d.h. gepreßte Pellets mit einem Durchmesser von weniger als 2,5 mm (Mikro-Tabletten), die zum Füllen von Kapseln (multi-unit-dose) geeignet sind.

Der einfache Granulier- und Verdichtungsvorgang dauert 5 bis 30, in der Regel 10 bis 15 Minuten. Während dieser Zeit soll die Temperatur des Produktes in den oben angegebenen Bereich steigen. Eine Wiederholung dieses Vorgangs mit dem erkalteten, so erhaltenen Granulat unter erneutem Zusatz von 2 bis 10 % Wasser wiederum bis zum Erreichen von 30 bis 55°C führt zur Bildung der erwähnten - im Vergleich zu üblichen - porenarmen Pellets mit glatter Oberfläche der Kügelchen und einer Schüttdichte von 0,5 bis 0,7, vorzugsweise 0,55 bis 0,65 g/ml und vor allem bisher unerreicht hohem Verapamil-Gehalt.

Die so hergestellten Pellets enthalten etwa 3 % Wasser. Dieses Wasser kann nach üblichen Trocknungsverfahren weitgehend (d.h. bis zu etwa ≦ 0,5 %) Restfeuchte entzogen werden. Solche Trocknungsverfahren sind in allen Handbüchern der pharmazeutischen Technologie beschrieben.

Die Art des Bindemittels ist unkritisch, es können alle pharmazeutisch geläufigen Bindemittel wie Gelatine, Stärke, Casein, Cellulosederivate (z.B. Methylcellulose, Hydroxypropylmethylcellulose), Schellack, Polyglykole und Polyvinylpyrrolidon in Mengen von weniger als 10, vorzugsweise weniger als 5, insbesondere weniger als 2 Gew.-%, bezogen auf Verapamil, eingesetzt werden.

Statt eines Bindemittels oder zusätzlich dazu (bis zu einer Gesamt-Hilfsmittelmenge von max. 10 Gew.-%) kann auch ein Retardierungsmittel eingesetzt werden.

Als Hilfsstoffzusätze, ggf. neben einem Binde- und/oder Retardiermittel, genügen für den Tablettenkern, falls überhaupt erforderlich, geringe Mengen pharmazeutisch üblicher Gleit- und Formentrennmittel wie Magnesiumstearat, Stearinsäure, Glyceroltribehenat, Aerosil und Talkum.

Zum Zweck einer Retardierung werden die Pellets oder Tabletten vorzugsweise mit einem retardierenden Film überzogen (lackiert). Man kann aber auch beim Granulieren geringe Mengen (max. 10 Gew.-%) Retardierungsmittel einbringen. Als Material für retardierende Filmüberzüge oder zum Einarbeiten in die Tablettenmasse kommen die üblichen in Betracht, wie sie in pharmakologischen Lehrbüchern beschrieben sind, beispielsweise Cellulosederivate und Copolymerisate von Acrylsäureestern und deren kationischen und anionischen Derivaten (®Eudragit E, R und S). Sie werden trocken, als organische Lösung oder vorzugsweise als wäßrige Dispersion aufgebracht.

Die wesentlichen Vorteile der erfindungsgemäßen Granulate und Pellets seien noch einmal kurz zusammengefaßt:

1. Sie haben eine weitgehend einheitliche Korngröße.

2. Sie besitzen eine hohe Festigkeit, so daß sie ohne Abrieb nach üblichen Verfahren zu pharmazeutischen Darreichungsformen weiterverarbeitet werden können.

3. Durch ihren niedrigen Gehalt an Hilfsstoffen und Zuschlägen bzw. durch deren Fehlen können aus ihnen kleine und kompakte (und somit für den Patienten leicht einzunehmende) Tabletten, Filmtabletten, Dragees oder Kapselfüllungen hergestellt werden.

4. Die Pellets sind weitgehend kugelförmig mit glatter und porenarmer Oberfläche, so daß sie mit sehr wenig Lack gut reproduzierbar retardiert werden können.

Beispiel 1 (Granulat-Herstellung)

2 kg gepulvertes Verapamil wurden in einem kleinen Hochleistungsgranulator mit Doppelmantel (Stephan Mischer, UMC 12, Stephan & Söhne, Hameln, D) auf 40°C erwärmt. Unter maximalen Energieeintrag wurden 100 g auf 40°C erwärmtes Wasser innerhalb von 30 sec zugegeben und solange (ca. 5 min) nachgranuliert, bis die Temperatur auf 50°C angestiegen war. Das abgekühlte Primärgranulat hatte folgende Korngrößenverteilung:

< 100 μm < 5 %
< 200 μm < 13 %
< 430 μm > 70 %
< 630 μm > 85 %
< 750 μm > 99 %.

Es wurde problemlos durch eine oszillierende Siebmaschine (Frewitt S.A., Fribourg, CH) mit 1,0 mm Siebeinsatz aufgelockert. Nach Trocknen im Umluft-Trockenschrank bei 40°C auf 0,4 % Restfeuchte wurde das Granulat ohne Schwierigkeiten durch eine oszillierende Siebmaschine mit 0,63 mm Siebeinsatz passiert; es trat kein Verschmieren der Siebmaschen auf. Die Rieselfähiigkeit, gemessen im DIN-Becher Nr. 8, betrug 305 g/min.

Nach Untermischem von 2 Gew.-% Talkum und 1 Gew.-% Magnesiumstearat wurde das Granulat zu mechanisch stabilen, leicht mit Lackdispersionen zu überziehenden Tabletten gepreßt, wobei deine Klebetendenz der Preßlinge an Matrizenwand oder Stempelflächen beobachtet wurde.

Mit dem durch ein 500 μm-Sieb gesiebten Granulat wurden Mikrotabletten mit 2 mm Durchmesser und 2 mm Höhe bei fast kugelförmiger Wölbung gepreßt, die mechanisch sehr fest und abriebarm waren, und die sich in der Wirbelschicht und auch in einem Lochtrommelcoater mit relativ wenig diffusionskontrollierendem Lack sehr wirksam retardieren ließen.

Vergleichsbeispiel

Granuliert man 2 kg Verapamil in dem kleinen Hochleistungsgranulator von Beispiel 1 bei üblichen Bedingungen, also bei Raumtemperatur, mit reinem Wasser, so werden zur gleichmäßigen Befeuchtung ca. 300 g benötigt. Das entstehende Granulat kann nicht durch ein 1,0 mm-Sieb passiert werden, da der Versuch zum Verschmieren und Verstopfen der Siebmaschinen führt. Auch nach Feuchtsieben über ein 3 mm-Sieb und üblichem Trocknen kann das Granulat nur schwer durch ein 1 mm-Sieb passiert werden, da auch das trockene Granulat zum Schmieren auf dem Sieb tendiert. Ein feines Granulat ist nicht herstellbar.

Der Versuch, ein derartiges Granulat unter Zusatz geringer Mengen an Tablettierhilfsstoffen (2 % Talkum, 1 % Magnesiumstearat) zu verpressen, scheitert, da die Masse unter Druck zum sofortigen Kleben an Matrizenwand und Stempeln führt. Entstehende Preßlinge haben auch keine Bindung, sie können zwischen Daumen und Zeigefinger zerdrückt werden. Pharmazeutisch einwandfreie Tabletten konnten erzeugt werden durch Eingranulieren von 20 % Lactose und 5 % Polyvinylpyrrolidon als Bindemittel in der Granulierflüssigkeit, jeweils bezogen als Massenanteil an der Granulattrockenmasse. Zum Verpressen war es darüber hinaus notwendig, einen Anteil von 5 % mikrokristalliner Cellulose in die Preßmasse einzumischen, um die Klebetendenz zu reduzieren. Mit diesem konventionellen Granulat war es jedoch nicht möglich, Mikropreßlinge von 2 mm Durchmesser herzustellen, da die Korngröße nicht genügend abgebaut werden konnte, und weil das Granulat bei den kleinen Stempeln nach kurzer Preßzeit zum Kleben tendierte, so daß beim Auseinanderfahren der Preßwerkzeuge die Preßlinge zum Teil auseinandergerissen wurden.

Beispiel 2 (Pellet-Herstellung)

In einen 50 1-Pharmamischer mit Horizontalpropeller und Zerhacker (Hersteller: Fa. Diosna, Osnabrück) wurden 10 kg Verapamilhydrochlorid eingewogen. Es wurden 0,5 kg Wasser von 27°C bei laufendem Mischwerkzeug dazugegebenen. Der Verfahrensablauf ergibt sich aus dem folgenden Protokoll:

| Vorgang | Dauer [min] | Mischer Stufe | Zerhacker Stufe | Endtemp. [°C] |
|---|---|---|---|---|
| Start | 0 | - | - | 21,5 |
| Wasserzugabe | 1 | 2 | 1 | 22,2 |
| Mischen | 20 | 2 | 2 | 45,0 |
| Abkühlen | 60 | - | - | 23,0 |
| Wiederholen des Befeuchtungs- und Mischvorganges | | | | |

Der Feuchtigkeitsgehalt der Pellets betrug bei Prozeßende 3,0 %. Die Trocknung auf 0,3 % Restfeuchte erfolgte im Umluft-Trockenschrank auf Hordenblechen bei 40°C Zulufttemperatur.

Die Rieselfähigkeit der Pellets wurde nach dem Trocknen gemessen. Die Pellets flossen aus einem DIN-Becher Nr. 8 in einer Zeit von 14-15 sec aus (entsprechend 235-250 g/min).

Die Korngröße der Pellets lag zu 80 % zwischen 0,5 und 0,8 mm. Die Schüttdichte der Pellets betrug 0,58-0,60 g/ml.

**Patentansprüche**

**Patentansprüche für folgende Vertragsstaaten : AT, BE, CH, DE, FR, GB, IT, LI, NL, SE, DK**

1. Feste Arzneiform mit einem Verapamilhydrochlorid-Gehalt von mindestens 90 Gew.-%.

2. Feste Arzneiform mit einem Verapamilhydrochlorid-Gehalt von mindestens 95 Gew.-%.

3. Feste Arzneiform mit einem Verapamilhydrochlorid-Gehalt von mindestens 98 Gew.-%.

4. Verapamilhydrochlorid-Granulat nach einem der Ansprüche 1 bis 3, bei dem mindestens 70 Gew.-% in einer Korngrößen-Bandbriete von 430 µm innerhalb eines Korngrößenbereichs von 0,1 bis 1 mm vorliegen,

4

und mit so guter Rieselfähigkeit, daß durch den DIN-Becher Nr. 8 mindestens 250 g/min hindurchlaufen.

5. Verapamilhydrochlorid-Pellets nach einem der Ansprüche 1 bis 3 mit einer Korngröße im Bereich von 0,3 bis 1,5 mm, wobei mindestens 70 Gew.-% im Bereich von 0,5 bis 1 mm liegen, und einer Schüttdichte von 0,5 bis 0,7 g/ml.

6. Verfahren zur Herstellung von Verapamilhydrochlorid-Granulat nach einem der Ansprüche 1 bis 4 durch Mischen von Verapamilhydrochlorid-Pulver mit Wasser oder einer wäßrigen Lösung oder Suspension eines Binde- oder Retardierungsmittels, dadurch gekennzeichnet, daß man Verapamilhydrochlorid-Pulver mit 0 bis 10 Gew.-% üblicher galenischer Hilfsstoffe und 2 bis 10 Gew.-% Wasser oder der genannten wäßrigen Lösung oder Suspension so intensiv mischt und verdichtet, daß die Produkttemperatur auf 30 bis 55°C steigt, und trocknet.

7. Verfahren nach Anspruch 6, dadurch gekennzeichnet, daß man so intensiv mischt und verdichtet, daß die Produkttemperatur auf 40 bis 50°C steigt.

8. Verfahren zur Herstellung von Verapamilhydrochlorid-Pellets nach einem der Ansprüche 1-5, dadurch gekennzeichnet, daß man zunächst nach dem Verfahren von Anspruch 6 oder 7 ein Granulat herstellt und dieses nach dem Erkalten noch einmal mit 2 bis 10 Gew.-5 Wasser befeuchtet und gemäß Anspruch 6 oder 7 behandelt.

9. Retardierende oder nichtretardierende Tablette, die aus einem Granulat nach Anspruch 4 oder aus Pellets nach Anspruch 5 gepreßt wurde.

10. Kapsel, die mit nach Anspruch 8 hergestellten retardierend oder nichtretardierend Pellets gefüllt ist.

**Patentansprüche für folgende Vertragsstaaten : GR, ES**

1. Verfahren zur Herstellung von Verapamilhydrochlorid-Granulat durch Mischen von Verapamilhydrochlorid-Pulver mit Wasser oder einer wäßrigen Lösung oder Suspension eines Binde- oder Retardierungsmittels, dadurch gekennzeichnet, daß man Verapamilhydrochlorid-Pulver mit 0 bis 10 Gew.-% üblicher galenischer Hilfsstoffe und 2 bis 10 Gew.-% Wasser oder der genannten wäßrigen Lösung oder Suspension so intensive mischt und verdichtet, daß die Produkttemperatur auf 30 bis 55°C steigt, und trocknet.

2. Verfahren nach Anspruch 1, dadurch gekennzeichnet, daß man so intensiv mischt und verdichtet, daß die Produkttemperatur auf 40 bis 50°C steigt.

3. Verfahren zur Herstellung von Verapamilhydrochlorid-Pellets nach Anspruch 1, dadurch gekennzeichnet, daß man zunächst nach dem Verfahren von Anspruch 1 oder 2 ein Granulat herstellt und dieses nach dem Erkalten noch einmal mit 2 bis 10 Gew.-% Wasser befeuchtet und gemäß Anspruch 1 oder 2 behandelt.

**Claims**

**Claims for the following Contracting States : AT, BE, CH, DE, FR, GB, IT, LI, NL, SE, DK**

1. A solid drug form containing not less than 90 % by weight verapamil hydrochloride.

2. A solid drug form containing not less than 95 % by weight verapamil hydrochloride.

3. A solid drug form containing not less than 98 % by weight verapamil hydrochloride.

4. Verapamil hydrochloride granules as claimed in any of claims 1 to 3, of which not less than 70 % by weight are within a 430 $\mu$m-wide band within a particle size range of from 0.1 to 1 mm, and with flowability such that not less than 250 grams flow through a DIN No. 8 cup in one minute.

5. Verapamil hydrochloride pellets as claimed in any of claims 1 to 3, with a particle size in the range from 0.3 to 1.5 mm, not less than 70 % by weight being in the range from 0.5 to 1 mm, and a bulk density of from 0.5 to 0.7 g/ml.

6. A process for producing verapamil hydrochloride granules as claimed in any of claims 1 to 4 by mixing verapamil hydrochloride powder with water or an aqueous solution or suspension of a binder or release-delaying agent, which comprises verapamil hydrochloride powder being so vigorously mixed with from 0 to 10 % by weight of conventional pharmaceutical auxiliaries and from 2 to 10 % by weight of water or the said aqueous solution or suspension and compacted that the product temperature rises to from 30 to 55°C, and dried.

7. A process as claimed in claim 6, with such vigorous mixing and compaction that the product temperature rises to from 40 to 50°C.

8. A process for producing verapamil hydrochloride pellets as claimed in any of claims 1-5, which comprises granules being initially produced by the process of claim 6 or 7 and, after cooling, being once again moistened with from 2 to 10 % by weight of water and treated as claimed in claim 6 or 7.

9. A tablet, with or without delayed release, compressed from granules as claimed in claim 4 or from pellets as claimed in claim 5.

10. A capsule which is filled with pellets, with or without delayed release, produced as claimed in claim 8.

**Claims for the following Contracting States : GR, ES**

1. A process for producing verapamil hydrochloride granules by mixing verapamil hydrochloride powder with water or an aqueous solution or suspension of a binder or release-delaying agent, which comprises verapamil hydrochloride powder being so vigorously mixed with from 0 to 10 % by weight of conventional pharmaceutical auxiliaries and from 2 to 10 % by weight of water or the said aqueous solution or suspension and compacted that the product temperature rises to from 30 to 55°C, and dried.

2. A process as claimed in claim 1, with such vigorous mixing and compaction that the product temperature rises to from 40 to 50°C.

3. A process for producing verapamil hydrochloride pellets as claimed in claim 1, which comprises granules being initially produced by the process of claim 1 or 2 and, after cooling, being once again moistened with from 2 to 10 % by weight of water and treated as claimed in claim 1 or 2.

**Revendications**

**Revendications pour les Etats contractants suivants : AT, BE, DE, FR, GB, IT, LI, NL, SE, DK**

1. Forme médicamenteuse solide d'une teneur en chlorhydrate de Verapamil d'au moins 90 % en poids.

2. Forme médicamenteuse solide d'une teneur en chlorhydrate de Verapamil d'au moins 95 % en poids.

3. Forme médicamenteuse solide d'une teneur en chlorhydrate de Verapamil d'au moins 98 % en poids.

4. Granulés de chlorhydrate de Verapamil selon une des revendications 1 à 3, dont au moins 70 % en poids sont d'une dimension de grain de 0,1 à 1 mm dans un intervalle des dimensions de grains de 430 µm, et fluides au point de traverser le becher DIN No. 8 à un débit d'au moins 250 g/min.

5. Pellets de chlorhydrate de Verapamil selon une des revendications 1 à 3, d'une dimension de grain dans l'intervalle de 0,3 à 1,5 mm dont au moins 70 % en poids dans l'intervalle de 0,5 à 1 mm, et une densité apparente de 0,5 à 0,7 g/ml.

6. Procédé pour la fabrication de granulés de chlorhydrate de Verapamil selon l'une des revendications 1 à 4 par mélange de chlorhydrate de Verapamil en poudre avec de l'eau ou une solution ou suspension aqueuse d'un liant ou retardateur, caractérisé en ce que l'on mélange et comprime le chlorhydrate de Verapamil en poudre avec 0 à 10 % en poids de produits auxiliaires galéniques usuels et 2 à 10 % en poids d'eau ou de la solution ou suspension auqueuse avec une énergie suffisante pour que le produit soit porté à une température de 30 à 55 degrés C et sèche.

7. Procédé selon la revendication 6, caractérisé en ce que l'on mélange et comprime avec une énergie suffisante pour que le produit monte à une température de 40 à 50 degrés C.

8. Procédé pour la fabrication de pellets de chlorhydrate de Verapamil selon une des revendications 1 à 5, caractérisé en ce que l'on prépare d'abord des granulés par le procédé de la revendication 6 ou 7 et, après refroidissement, on humidifie encore une fois ces granulés par 2 à 10 % en poids d'eau et on les traite selon la revendication 6 ou 7.

9. Comprimés à retard ou non, formés à la presse à partir de granulés selon la revendication 4 ou de pellets selon la revendication 5.

10. Capsule qui est garnie de pellets à retard ou non, préparée selon la revendication 8.

**Revendications pour les Etats contractants suivants : GR, ES**

1. Procédé pour la fabrication de granulés de chlorhydrate de Verapamil par mélange de chlorhydrate de Verapamil en poudre avec de l'eau ou une solution ou suspension aqueuse d'un liant ou retardateur, caractérisé en ce que l'on mélange et comprime du chlorhydrate de Verapamil en poudre avec 0 à 10 % en poids de produits auxiliaires galéniques usuels et 2 à 10 % en poids d'eau ou de la solution ou suspension aqueuse avec une énergie suffisante pour que le produit monte à une température de 30 à 55 degrés C et sèche.

2. Procédé selon la revendication 1, caractérisé en ce que l'on mélange et comprime avec une énergie suffisante pour que le produit monte à une température de 40 à 50 degrés C.

3. Procédé pour la fabrication de pellets de chlorhydrate de Verapamil selon revendication 1, caractérisé en ce que l'on prépare d'abord des granulés par le procédé de la revendication 1 ou 2 et, après refroidissement, on les humidifie encore une fois par 2 à 10 % en poids d'eau et on les traite selon la revendication 1 ou 2.